# EUROPEAN PATENT SPECIFICATION

(11) **EP 3 349 641 B1**
(45) Date of publication and mention of the grant of the patent: **10.09.2025**
(21) Application number: 16809517.2
(22) Date of filing: 15.09.2016
(51) Int. Cl.: A61B 1/307, A61M 3/02

(54) **ADAPTER AND ASSEMBLY FOR INSTILLATION OF AGENTS INTO THE BLADDER THROUGH THE URETHRA WITHOUT CATHETER**
ADAPTER UND ANORDNUNG ZUR INSTILLATION VON WIRKSTOFFEN IN DIE HARNBLASE DURCH DIE HARNRÖHRE OHNE KATHETER
ADAPTATEUR ET ENSEMBLE POUR INSTILLATION D'AGENTS DANS LA VESSIE PAR L'URÈTRE SANS CATHÉTER

(30) Priority: 16.09.2015 HU 1500419; 23.12.2015 HU 1500648
(43) Date of publication of application: 25.07.2018
(73) Proprietor: Lovász, Sándor, 1136 Budapest (HU); Rényi, Gábor, 1137 Budapest (HU); Giber, János, 1122 Budapest (HU)
(72) Inventor: Lovász, Sándor, 1136 Budapest (HU); Rényi, Gábor, 1137 Budapest (HU); Giber, János, 1122 Budapest (HU)
(74) Representative: Giber, Janos
(86) International application number: PCT/HU2016/000063
(87) International publication number: WO 2017/046621

(56) References cited:
- EP-A2- 0 726 079
- WO-A1-2013/050404
- CN-U- 203 663 226
- CN-Y- 201 248 960
- KR-Y1- 200 388 699
- LU-A1- 50 293
- US-A- 1 720 198
- US-A- 4 776 848
- US-A1- 2009 054 876
- US-A1- 2013 204 089

## Description

The subject matter of our invention is an adapter, advantageously single use, advantageously cylindrical, advantageously syringe adapter, made out of solid, pharmaceutically applicable, advantageously for human therapy applicable, advantageously rigid or flexible material, for injection of active and/or contrast agent in liquid, fluid-like, gas or colloid disperse form, painlessly or by reduced pain, without leakage into the urethra or into the urethra and the bladder through the external urethral orifice without using catheter.

The 6 adapter is provided with a 5 central, longitudinal, inner through hole and has a rounded off 1 tip-part to be inserted into the external urethral orifice, a 2 sealing collar and a 3 cylindrical connecting part known connectable to the 4 appropriate part of a dosing device, advantageously to the ISO 594 standard Luer Slip or Luer-Lock tip of a syringe.

The form of the end of the 1 tip-part of the adapter according to the invention inserted into the external urethral orifice is specially rounded off, softened, advantageously cylindrical, further advantageously conical, and is characteristic for the invention and has unique parameters.

Furthermore the adapter according to the invention fits generally and perfectly to the external urethral orifice.

The material of the adapter according to the invention is solid advantageously rigid or flexible, pharmaceutically applicable advantageously for human therapy applicable material, especially advantageously metal which may be especially advantageously copper, bronze, aluminum and stainless steel or any alloy of them, further advantageously glass, further advantageously plastic, which may be especially advantageously thermosetting, thermoplastic or flexible plastic, thermoset elastomer, especially advantageously silicone, thermoplastic elastomer, especially advantageously thermoplastic polyurethane, further advantageously synthetic rubber or synthetic rubber like material, or synthetic rubber or synthetic rubber like based material, especially advantageously polytetrafluoroethylene (Teflon, PTFE), polyethylene, polypropylene, polystyrene, polyester, polylactic acid, polycarbonate, polyvinyl chloride, polyethersulfone, polyacrylate, hydrogel (acrylate), polysulfone, polyetheretherketone (PEEK), poly-p-xylylene (parylene), fluoropolymer, further advantageously any natural based rigid or flexible material, especially advantageously latex or latex based, or natural based rubber or rubber like material or rubber or rubber like material based material, further especially advantageously plastic or alloy or mixture of any of the above listed materials, further advantageously any other pharmaceutically applicable advantageously for human therapy applicable, for mass producing, for quantity production in series suitable material and by technology for mass producing, for quantity production especially advantageously by die casting or injection molding technology, or 3D printing in series producible material, which can be used for producing the adapter according to the invention.

Using the adapter according to the invention, the subject matter of our invention is furthermore a non-invasive, humane, catheter-free, leakage-free process
for injection of active and/or contrast agent in liquid, fluid-like, gas or colloid disperse form, advantageously in aerosol, emulsion, gel, foam or suspension form
into the urethra or into the urethra and the bladder through the external urethral orifice
from dosing device, advantageously syringe, by using overpressure, advantageously by using manual or gravity pressure, without using catheter.

By the process according to the invention the 6 adapter is connected by known method to the 4 appropriate part of a dosing device containing the active and/or contrast agent, advantageously to the ISO 594 standard Luer Slip or Luer-Lock tip of a syringe, then the active and/or contrast agent is injected leakage-free into the urethra or into the urethra and the bladder through the adapter, then through the external urethral orifice by using overpressure in the dosing device and inserting the 1 tip-part into the external urethral orifice and squeezing the 2 sealing collar in a proper way to the external urethral orifice.

The process according to the invention is for both genders adults and children, advantageously for women, men and children applicable.

The dosing device used in the process according to the invention can be used for injection active and/or contrast agents for local treatment or for diagnosis of bladder and/or urethra by using overpressure into the urethra and the bladder through the external urethral orifice.

Using the adapter and process according to the invention, catheter-free, painless or pain-reduced and leakage-free advantageously bladder filling, bladder rinsing, bladder diagnosis, especially advantageously intravesical (bladder) instillation can be implemented.

According to the invention by the injection of the active and/or contrast agents into the bladder through the external urethral orifice and urethra by using the adapter and catheter-free process according to the invention, direct local treatment and diagnosis of the urethra can be implemented, which is impossible by using catheter.

Furthermore the subject matter of the invention is the use of the adapter and process according to the invention for injection of active and/or contrast agent in liquid, fluid-like, gas or colloid disperse form, painlessly or by reduced pain, without leakage into the urethra or into the urethra and the bladder through the external urethral orifice without using catheter.

During the use according to the invention, the injected active agent is advantageously solution of curative active ingredient or of drug, or a mixture of them, the so called "bladder cocktail" for local, advantageously local curative or pharmaceutical treatment of bladder and/or urethra.

The adapter and catheter-free process according to the invention can be used advantageously for painless or pain-reduced bladder filling, bladder rinsing, especially advantageously for intravesical (bladder) instillation, namely for the local pharmaceutical painless or pain-reduced treatment of the bladder and further especially advantageously for the local painless or pain-reduced pharmaceutical treatment of the urethra.

The adapter and process according to the invention can be used further advantageously for injection of agent in liquid, fluid-like, gas or colloid disperse form especially advantageously in liquid form, comprising active and/or contrast agent, advantageously solution of curative active ingredient or drug, further advantageously diagnostic solution comprising contrast agent,
through the external urethral orifice and urethra into the bladder,
advantageously for local treatment of bladder and/or urethra especially advantageously local treatment of interstitial cystitis, bladder pain syndrome, further especially advantageously with analgesic effect; further especially advantageously for local anesthesia, further especially advantageously for local treatment of low urinary tract symptoms, further especially advantageously for local analgesic after-treatment of patients irradiated for prostate cancer, bladder cancer or pelvis tumor, further especially advantageously for local chemotherapeutical treatment of female patients suffering in bladder cancer

Using the adapter and process according to the invention the agent can be injected safe, catheter- and leakage-free for the above indications, advantageously for local treatments and diagnostic targets.

### The state of prior art practice and science

The generally accepted and widespread practice in cases of various diseases of the bladder is direct injection of the solution with curative effect, or solution of drug or mixture of these solutions (so called "bladder cocktails") directly into the bladder generally through a catheter introduced into the urethra. One of the target of the treatment is to reproduce and heal the so called "GAG" (glycosaminoglycan) layer, which is a defending mucous-layer on the surface of the inner wall of the bladder.

Thus the active ingredients arrive into the bladder in higher concentration than being orally or parenterally administered bypassing the intestinal tract by injection. As only a fraction of the administered dose of drug is absorbed and gets into the circulation in this way the risk of emerging undesirable or even dangerous side effects can also be minimized.

For the local treatment of the bladder (bladder-instillation) with curative active ingredients or drugs, all accessible special medical literature, professional guidelines as well as textbooks recommend the method with catheter as the only possible method.

This generally accepted method however has several significant disadvantages:
Prior to applying the method with catheter a lubricant gel has to be applied rendering the method more expensive and the process more complicated. In more than half of the cases of interstitial cystitis (bladder pain syndrome) the urethra itself is also involved in the disease and therefore it is hypersensitive for contacting or pressing it and often it can even cause expressed pain, making patients' sexual life intolerable as well as sitting and walking. In such cases applying the catheter in the urethra may cause severe pain resulting in refusal of treatment..

The straight and relatively inflexible draining catheters straighten the bends of the urethra thereby scraping along one or the other side of the surface of the inner wall of the urethra, causing minimally microscopic superficial mucous membrane lesions but sometimes also major lesions with macroscopic bleeding (urethrorrhagia) can be seen.

Due to the anatomic differences between the genders, the longer urethra in men with several natural bends is even more frail and during the course of the treatment (lasting 10 to 15 weeks with regular, weekly one catheterization) the repeated iatrogenic lesions at the same points of the urethra may even result urethral stricture caused by scar tissue as tardive complication.

The drugs administered by catheter remain until the following urination in the bladder because in the moment of pulling out the catheter, the natural muscular closing system of the urethra (internal and external sphincter) closes and therefore no active ingredient gets into the urethra.

Therefore the task of the invention is to develop a method by which the solution comprising the active ingredient can be injected into the bladder without catheterization of the urethra and at the same time a smaller quantity of drug, but with effective concentration can remain in the urethra. Direct injection into the urethra seemed to be a good solution. However regarding the difference between the internal diameter of the external urethral orifice and the outside diameter of the Luer-lock nozzle, tip of the syringe used for injection, a significant loss of liquid can occur by leakage hindering patients of both genders being such interventions successful.

The adequate application of the adapter according to the invention provides a solution of these troubles and difficulties.

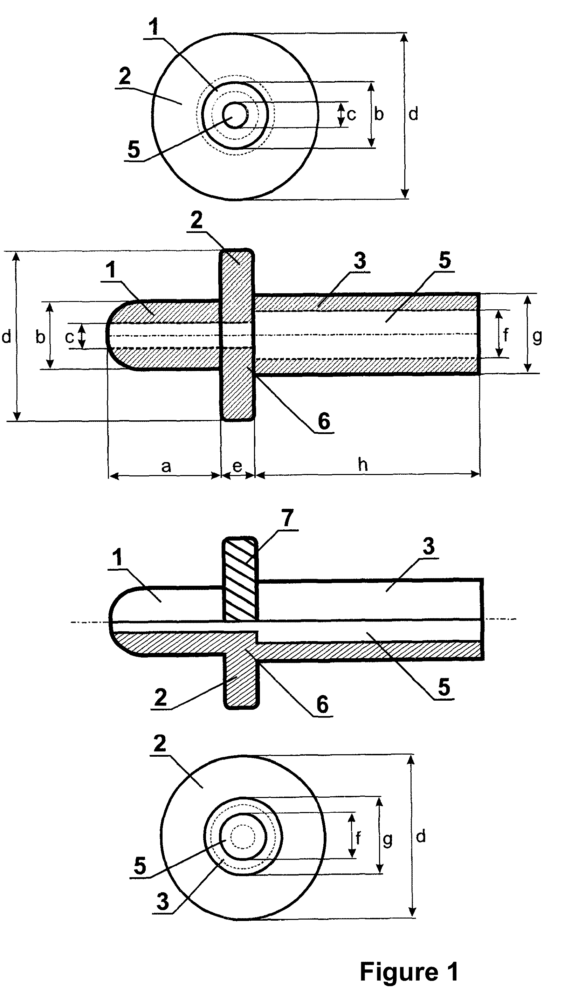

### The specification of the adapter according to the invention according to the Figures 1 to 3

According to the definitions and notations of the Figures 1 to 3, the adapter according to the invention can be described as follows.

In Figure 1 enclosed to the specification, the 6 adapter according to the invention is presented in three views, namely top-view, side-view and bottom view.

The side view is presented in two versions:
in a basic segment version and in a half view - half segment version.

The 6 adapter according to the invention has three parts as clearly shown in the side view of Figure 1:
the 1 tip-part fitting directly to the urethra through the external urethral orifice,
the 2 sealing collar preventing leakage of agent, advantageously liquid injected into the urethra
and the 3 cylindrical connecting part , connecting to the 4 appropriate part of the dosing device, advantageously to one of the type of ISO 594 standard Luer Taper tip, either to Luer Slip or to Luer-Lock tip of the syringe by known method, advantageously precisely connected or built on.

Concerning Luer Slip fitting, the 3 cylindrical connecting part of the adapter is connected by a standard conic tip, concerning Luer Lock fitting, the connection is additionally also fixed by thread or a bayonet lock in case of high pressure injection.

As in our case low pressure is used, according to the solution of our invention, such a fixation is not needed even concerning Luer Lock fitting, so the 3 cylindrical connecting part can be simply fitted to the Luer Lock tip also by pressing together and held by friction similar to Luer Slip fitting.

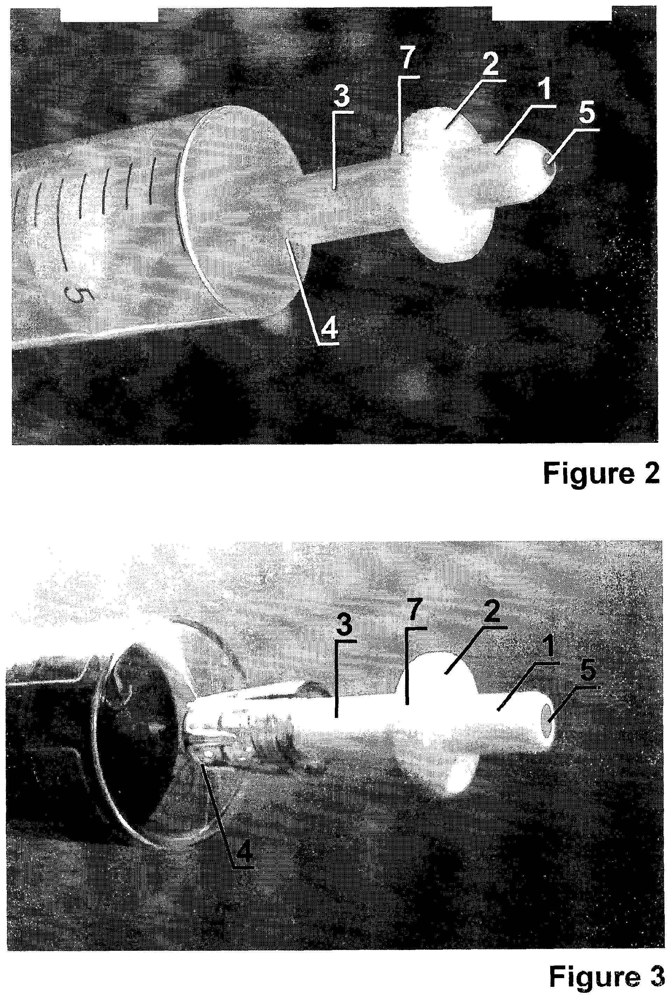

In Figure 2 Luer Slip fitting and in Figure 3 Luer Lock fitting are presented, and is also shown and signed in both Figures, that according to the solution of the invention the external circumference of the 2 sealing collar is 7 knurled to facilitate adjusting and removal to the external urethral orifice by hindering the slipping.

As clearly shown in all three views of Fig. 1, the 6 adapter according to the invention is provided with a 5 central, longitudinal inner through hole through which the agent, advantageously the liquid gets from the dosing device, advantageously from liquid dosing device, advantageously from syringe, further advantageously from infusion device through the 1 tip part and 3 cylindrical connecting part into the external urethral orifice and then into the urethra. The parts of 5 central, inner through hole of 1 tip-part and of 3 cylindrical connecting part of the 6 adapter according to the invention have advantageously different "c" and "f" diameters.

An important characteristic of the adapter according to the invention and also presented in top and side view of Fig. 1 is, that the 1 tip-part to be inserted into the orifice of the urethra is rounded off specially, its surface, and edges are softened, thus the inner wall of the urethra would be not injured even if a precise coaxial insertion cannot be achieved.

The characteristic sizes of the different parts of the adapter according to the invention are signed by small letters in the figures.

According to the side view of Fig. 1 the "a" length of the 1 tip-part to be inserted into external urethral orifice is advantageously 8 to 12 mm especially advantageously 10 mm to assure safe fitting and its "b" diameter is advantageously 6 mm (18 French) corresponding to an average catheter diameter and can be inserted in the overwhelming majority of cases without difficulty into the urethra.

As can be seen in the top view of Fig. 1 the advantageous "c" diameter of the 5 central, longitudinal inner through hole of the 1 tip-part to be inserted into the urethra is advantageously 1,8 mm to 3,5 mm, especially advantageously 2,5 mm so that a thick gel-like solution or suspension can be injected with small resistance into the urethra.

A further characteristic of the invention is, that the edges of the tip of the hole is softened by fine machining.

The end of the 1 tip-part according to the solution of the invention has a softened shape, so, that no sharp edges causing mucosal lesion should remain. The round-off radius at the 1 tip part's end is no single data, its tolerance is 1mm downwards (smaller) or 2mm upwards (bigger). The round off and softening are also applied outward and inward to the 5 central, inner through hole.

According to the top view and bottom view of Figure 1, the 2 sealing collar of the adapter according to the invention is a disk. The "d" diameter of the disk is advantageously 12 mm to 20 mm, specially advantageously 15 mm and the "e" thickness of the disk is advantageously 2 mm to 5 mm especially advantageously 3 mm as illustrated in the side view of Figure 1. The disk fits over the roughly round or oval shaped external urethral orifice, and assures perfect sealing without leakage.

The mucous surfaces close to the external urethral orifice are sensitive even to microscopic lesion, therefore the edges of the disk as a solution by the invention are chamfered to 45° or rounded off in a circle advantageously with 1 mm radius and the remained edges of the disk should also be softened.

According to the solution of the invention, as shown in the half-segment-half view version of side view in Figure 1 and in Figures 2 and 3, presenting the Luer Tapers, the external circumference of the 2 sealing collar is 7 knurled to facilitate adjusting and removal to the external urethral orifice by hindering the slipping.

According to the bottom view of Figure 1 and side-view of Figure 1, the 5 central, longitudinal internal hole of 6 adapter is shown as hole of 3 cylindrical connecting part. The "f" inner caliber of the 5 central, inner through hole of 3 cylindrical connecting part is advantageously 3,8 mm to 5,2 mm especially advantageously 4,2 mm and "g" outer diameter is advantageously at least 7 mm, specially advantageously 7 mm which characteristic parameters assure the thickness of wall of cylinder for suitable stability.

According to the side view of Fig. 1 the "h" length of the 3 cylindrical connecting part is advantageously at least 20 mm, especially advantageously 20 mm, to assure the required visual control for the person performing the treatment so even dosing devices advantageously syringes with larger diameter (with e.g. 50 ml capacity) cannot hide the adapter being inserted into the external urethral orifice.

The 3 cylindrical connecting part of the 6 adapter according to the invention is connected by known method, advantageously precisely connected, linked, integrated or built to the 4 appropriate part of the dosing device, advantageously to a syringe, especially advantageously to the ISO 594 standard Luer Slip or Luer Lock tip of the syringe, it can be also a part of the dosing device. The advantageous solutions are illustrated in Figures 2 and 3.

### Detailed specification of the process according to

### the invention

The essential of the catheter-free, for both genders, adults and children, advantageously for women, men and children applicable process according to the invention is, that after fitting, connecting or building the adapter according to the invention to the appropriate part of the dosing device advantageously to the ISO 594 standard Luer Slip or Luer-lock tip of syringe by known method, the active and/or contrast agent is injected leakage-free into the urethra and/or into the bladder through the external urethral orifice and the urethra, using overpressure, advantageously low overpressure, especially advantageously maximum pressure of 60 water cm for men, male patients and maximum pressure of 30 water cm for women, female patients

As an advantageous solution of the invention the active and/or contrast agent is injected, , advantageously slowly injected into the urethra and/or bladder through the external urethral orifice painlessly or by reduced pain, avoiding lesions of urethra, further advantageously injected using gravity pressure, especially advantageously using an infusion device, further advantageously pressing out from balloon or tube, using manual pressure by a method as above, so, that the active agent advantageously active agent with curative effect or drug, further advantageously diagnostic agent comprising contrast agent should remain in appropriate agent concentration of active ingredient or diagnostic agent also in the urethra in its full length.

The leakage-free, catheter-free process, injection is implemented by a method, that the adapter according to the invention fitted or connected by known method - defined and advantageously defined as above - to the dosing device or built on the dosing device advantageously to the syringe as above, is squeezed to the external urethral orifice, and during injection the leakage, flow is inhibited by the help of the 2 sealing collar of the 6 adapter.

A primary basic condition of the leakage-free, catheter-free process, injection, is the steady visibility of the adapter as placed in the urethral orifice whereby any leakage or flow can be detected in due time and eliminated by changing the axial direction and/or the force of squeeze. The visibility depends on the "h" size of characteristic length of the 3 cylindrical connection part (Figure 1, side-view), which size should be advantageously at least 20 mm, especially advantageously 20 mm.

Further condition of the leakage-free, catheter-free process is, that the dosing device, advantageously syringe or infusion device can be used for injection, of active and/or contrast agent advantageously agents defined as above into the bladder through the urethra for local treatment or diagnosis of bladder and/or urethra and also for ensuring of the appropriate overpressure.

In addition the process according to invention avoids the superficial lesions and bleedings of the urethra or the mucosa covering the surface of urethra and so the process for the patient is non-invasive painless or pain-reduced.

The prevention, reducing and avoiding the pain of urethra caused by the intervention itself, and reducing the fear of treatment at the same time results a better relaxation, which renders the treatment be better tolerable for the patient because reduced overpressure is needed for the injection of agent.

Further advantage of the catheter-free, non-invasive, humane, leakage-free process according to the invention is, that the agents listed above advantageously agents with curative effect or drug, further advantageously agent, which can be injected comprising diagnostic agent, advantageously liquid, advantageously solution evolve their effect in the urethra itself also, increasing the efficiency of the therapeutic process or diagnostic usage.

Based on the above the subject matter of our invention furthermore is a non-invasive, humane, catheter-free process
for painless or pain-reduced and leakage-free injection of active and/or contrast agent in liquid, fluid-like, gas or colloid disperse form, advantageously in aerosol, emulsion, gel, foam or suspension form
into the urethra or into the urethra and the bladder through the external urethral orifice by using overpressure, advantageously by using manual or gravity pressure in the dosing device, without using catheter.

During the process according to the invention the 3 connecting part of the 6 adapter is connected by known method to the 4 appropriate part of a dosing device, advantageously of a syringe, further advantageously to an infusion device containing the active and/or contrast agent, advantageously to the ISO 594 standard Luer Slip or Luer-Lock tip of a syringe, then the active and/or contrast agent is injected, advantageously slowly injected, leakage-free into the urethra or into the urethra and the bladder through the 6 adapter, then through the external urethral orifice by inserting the 1 tip-part of the adapter into the external urethral orifice and squeezing the 2 sealing collar of the adapter in a proper way to the external urethral orifice.

Using this method the therapeutic and diagnostic agents, defined and advantageously defined as above and as further on for the indications and treatments depending on the injected agents, defined and advantageously defined as above and as further on, can be injected painlessly or by pain-reduced into the urethra and/or into the bladder, without using catheter, any lesion, so that the agent, advantageously liquid or gas, or different agents of colloid disperse systems in appropriate concentration of active agent and/or diagnostic agent, advantageously contrast agent should remain also in the urethra in its full length, to evolve there also their therapeutic and diagnostic effect.

This causes gradually increase of the pressure in the urethra and when reaching a certain pressure (retrograde sphincter opening pressure) the sphincter opens and the agent, advantageously the liquid flows into the bladder. This pressure is about 60 cm water column for men, males, and for women, females the average pressure is 30 cm water column lower. These values can be reduced significantly by injection of, advantageously by slowly injection of the agent, advantageously liquid, advantageously solution with curative effect or drug solution, and so by avoiding pain in the urethra and reducing the fear of the treatment and by deliberate relaxation. Thus steadily low pressure (30 to 40 cm water column) expands the urethra to a significant lover extent causing less discomfort and pain.

### The applications according to the invention

The adapter and process according to the invention can be used advantageously for injection of active and/or contrast agents, especially advantageously solution of curative active ingredient or of drug, or of diagnostic agent, advantageously contrast agent into the urethra or into the urethra and the bladder through the external urethral orifice for local treatment of bladder and/or urethra, further advantageously for bladder filling, bladder rinsing, especially advantageously for intravesical (bladder) instillation or further advantageously for diagnosis.

Furthermore the adapter and process according to the invention can be used advantageously for injection active and/or diagnostic agent advantageously contrast agent in liquid, fluid-like, gas or colloid disperse form especially advantageously in aerosol, emulsion, gel, foam or suspension form into the urethra and/or bladder through the external urethral orifice for
local treatment of bladder and/or urethra especially advantageously local treatment of interstitial cystitis, bladder pain syndrome, further especially advantageously with analgesic effect, further especially advantageously for local anesthesia, further especially advantageously for local treatment of low urinary tract symptoms, further especially advantageously for local analgesic after-treatment of patients irradiated for prostate cancer, bladder cancer or pelvis tumor, further especially advantageously for local chemotherapeutical treatment of female patients suffering in bladder cancer.

Because of the short urethra the injection of active agents for local chemotherapeutical treatment of bladder can be used only in case of female patients. Because of the larger size of the urethra of male patients and the danger of lesion of the urethra this application cannot be used by men.

### Advantages

1. Using the adapter according to the invention by injection of the solution with curative effect or drug solution or diagnostic solution substantially reduces the inconveniences and pain brought about by the treatment.
2. The catheter-free injection, completely prevents larger and/or smaller mucosal lesions and bleedings of the urethra and serious iatrogenic lesions, as e.g. tardive urethral stricture or chronic bacterial urethritis can be prevented.
3. In the course of treatment only the sterile agent, advantageously solution gets into the urethra and into the bladder thus the risk of bacterial infection and of developing low urinary symptoms is reduced.
4. The process is simple, requires less equipment than bladder filling, bladder instillation with catheter, therefore the treatment without catheter is faster and is less expensive at the same time.
5. As the drug solution is injected into the bladder through the urethra therefore drug solution with unchanged concentration - here not diluted by the urine - remains also in the entire length of the urethra until the following urination (for 2 to 3 hours). -Therefore the treatment is more effective, the patients are getting sooner symptom-free and the diagnosis of any urethral irregularity or lesion gets easier.
6. The treatment or diagnosis of urethra is solely by the catheter-free process according to the invention is possible. By a process using a catheter the treatment of urethra is not possible at all.
7. The cost of production of the adapter and therefore its price is significantly lower than the common price of draining catheter plus of the lubricant gel for the insertion of catheter. Therefore the costs of a process without using catheter and also the gel for the catheter are significantly lower than the present practice using the process with catheter.

## Claims

1. Adapter (6) made of solid, pharmaceutically applicable material for intravesical instillation, injection of active and/or contrast agent in liquid, fluid-like, gas or colloid disperse form into the bladder through the urethra, ***characterized by***
- that the adapter (6) is for replacement of a catheter and suitable for painless, non-invasive leakage-free injection into the urethra and the bladder through the external urethral orifice of both male and female patient;
- the adapter (6) is provided with a central, longitudinal inner through hole (5) and
- with a distal end having a rounded off tip-part (1) configured to be inserted separately and directly into the external urethral orifice of both male and female patients;
- the distal end having a distal segment of the central, longitudinal inner through hole (5) with a constant "c" internal diameter and
- the adapter (6) is made of a flexible material and provided with a sealing collar (2) with a diameter larger than a diameter of the distal end wherein said sealing collar (2) fits over the roughly round or oval shaped external urethral orifice, and thereby assures perfect sealing without leakage and thereby creating overpressure to open the sphincter of the bladder in both male and female patients and
- the adapter (6) is provided with a proximal end having a cylindrical connecting part (3) connectable to the connecting part (4) of a dosing device, the cylindrical connecting part (3) having a proximal segment of the central, longitudinal inner through hole (5) with a constant "f" internal and "g" outer diameter;
- wherein the "f" inner diameter of the proximal segment of the central, longitudinal inner through hole (5) is larger than the "c" inner diameter of the distal segment of the central, longitudinal inner through hole (5) and the distal segment and the proximal segment of the central, longitudinal inner through hole (5) are in fluid communication;
- and wherein the "h" length of the proximal end of the connecting part (3) is at least 20 mm;
- wherein the "f" inner diameter of the proximal segment of the inner through hole (5) of the cylindrical connecting part (3) is 3,8 mm to 5,2 mm differing from the "c" inner diameter of the distal segment of the central, longitudinal inner through hole (5), and a "g" outer diameter of the cylindrical connecting part (3) is minimum 7 mm;
- with the distal segment with "c" diameter of the central, longitudinal inner through hole (5) being on one side of the flexible sealing collar (2) and the proximal segment with "f" diameter of the central, longitudinal inner through hole (5) being on the other side of the flexible sealing collar (2);
- and wherein the "a" length of the distal segment having a rounded off tip-part (1) from the sealing collar (2) to the rounded off tip-part (1) to be inserted into the external urethral orifice is 8 mm to 12 mm; and
- the "b" outer diameter of the distal end is 6 mm (18 French) corresponding to an average catheter;
- and wherein the "c" inner diameter of the distal segment of the central, longitudinal inner through hole (5) is 1,8 mm to 3,5 mm differing from the "f" inner diameter of the proximal segment of the inner through hole of the cylindrical connecting part (3);
- and wherein the flexible sealing collar (2) of the adapter has a "d" diameter of 12 mm to 20 mm, and an "e" thickness of 2 mm to 5 mm.

2. The adapter according to claim 1 ***characterized by**,* that the form of the end of the tip-part (1) is configured to be inserted into the external urethral orifice is cylindrical or conical.

3. The adapter according to any of claims 1 to 2 ***characterized by**,* that the adapter is a syringe adapter, and the dosing device is a syringe.

4. The adapter according to any of claims 1 to 3 ***characterized by**,* that the connecting part (4) of dosing device is the ISO 594 standard Luer Slip tip or Luer Lock tip of a syringe.

5. The adapter according to any of claims 1 to 4 ***characterized by**,* that the cylindrical connecting part (3) of the adapter (6) precisely fits, is connected, or built to the connecting part (4) of the dosing device or is part of the dosing device.

6. The adapter according to any of claims 1 to 5 ***characterized by**,* that the solid, pharmaceutically applicable material of the adapter is for human therapy applicable, flexible material and is plastic or natural based material or any alloy or mixture of them or
is thermosetting, thermoplastic or flexible plastic or thermoset elastomer or thermoplastic elastomer or latex or latex based, or synthetic or natural based rubber or rubber based or rubber like material or rubber like material based material or any mixture of them.

7. The adapter according to claim 6 ***characterized by**,* that the plastic the pharmaceutically applicable, solid material of the adapter is made of is: thermoplastic polyurethane, polytetrafluoroethylene (Teflon, PTFE), polyethylene, polypropylene, polystyrene, polyester, polylactic acid, polycarbonate, polyvinyl chloride, polyether-sulfone, polyacrylate, hydrogel (acrylate), polysulfone, polyetheretherketone (PEEK), poly-p-xylylene (parylene), fluoropolymer, or silicone or any mixture of them.

8. The adapter according to any of claims 6 to 7 ***characterized by**,* that the pharmaceutically applicable, solid material of the adapter is material for mass producing in series, suitable for die casting technology, or 3D printing.

9. The adapter according to any of claims 1 to 8 ***characterized by**,* that at the end of the tip-part (1) configured to be inserted into the external urethral, the tip of the inner through hole (5) are softened so that no sharp edges causing mucosal lesion should remain and the round off and softening are outward and inward towards to the inner through hole (5) achieved also and so the tip part (1) rounded of and softened fits perfectly and generally to the external urethral orifice of both male and female patients.

10. The adapter according to any of claims 1 to 9 ***characterized by**,* that the "a" length of tip part (1) configured to be inserted into the external urethral orifice is 8 mm or 10 mm, the "c" diameter of the central, longitudinal inner through hole (5) of the distal segment having the tip-part (1) is 2,5 mm or 3,5 mm.

11. The adapter (6) according to any of the claims 1 to 10 ***characterized by**,* that the "f" inner diameter of the proximal segment of the central longitudinal inner through hole (5) of the cylindrical connecting part (3) is 4,2 mm or 3,8 mm and the "g" outer diameter of the cylindrical connecting part (3) is 7 mm.

12. The adapter (6) according to any of the claims 1 to 11 ***characterized by**,* that the "h" length of the proximal segment having the cylindrical connecting part (3) is 20 mm.

13. The adapter according to any of claims 1 to 12 ***characterized by**,* that the "d" diameter of the flexible sealing collar (2) is 15 mm, and the "e" thickness is 2 mm or 3 mm.

14. The adapter according to any of claims 1 to 13 **characterized by**, that the edges of the sealing collar (2) are chamfered to 45° or rounded off in a circle with 1 mm radius and the remained edges are softened.

15. The adapter according to any of claims 1 to 14 **characterized by**, that the external circumference of the sealing collar (2) is knurled (7).

16. The adapter according to any of claims 1 to 15 ***characterized by**,* that it is for single use.

17. An assembly comprising a dosing device and the adapter (6) according to any of the preceding claims wherein the dosing device precisely fits, is connected, linked, integrated or built to the adapter (6) according to claims 1 and 5.

## Patentansprüche

1. Adapter (6), der aus festem, pharmazeutisch anwendbarem Material gefertigt ist, zur intravesikalen Instillation, Injektion von Wirkstoffen und/oder Kontrastmitteln in flüssiger, fluidähnlicher, gasförmiger oder kolloiddisperser Form in die Blase durch die Harnröhre hindurch, **dadurch gekennzeichnet,**
- **dass** der Adapter (6) als Ersatz für einen Katheter dient und für eine schmerzlose, nicht-invasive und leckagefreie Injektion in die Harnröhre und die Blase durch die äußere Harnröhrenöffnung hindurch sowohl bei männlichen als auch bei weiblichen Patienten geeignet ist;
- der Adapter (6) mit einem zentralen, länglichen inneren Durchgangsloch (5) bereitgestellt ist, und
- mit einem distalen Ende, das ein abgerundetes Spitzenteil (1) aufweist, das konfiguriert ist, um separat und direkt in die äußere Harnröhrenöffnung von sowohl männlichen als auch weiblichen Patienten eingeführt zu werden;
- das distale Ende ein distales Segment des zentralen, länglichen inneren Durchgangslochs (5) mit einem konstanten Innendurchmesser "c" aufweist, und
- der Adapter (6) aus einem flexiblen Material gefertigt ist und mit einer Dichtungsmanschette (2) mit einem Durchmesser bereitgestellt ist, der größer ist als ein Durchmesser des distalen Endes, wobei die Dichtungsmanschette (2) über die etwa runde oder oval geformte äußere Harnröhrenöffnung passt und dadurch eine perfekte Abdichtung ohne Leckage gewährleistet und dadurch einen Überdruck schafft, um den Schließmuskel der Blase sowohl bei männlichen als auch bei weiblichen Patienten zu öffnen, und
- der Adapter (6) mit einem proximalen Ende bereitgestellt ist, das ein zylindrisches Verbindungsteil (3) aufweist, das mit dem Verbindungsteil (4) einer Dosiervorrichtung verbindbar ist, wobei das zylindrische Verbindungsteil (3) ein proximales Segment des zentralen, länglichen inneren Durchgangslochs (5) mit einem konstanten Innendurchmesser "f" und Außendurchmesser "g" aufweist;
- wobei der Innendurchmesser "f" des proximalen Segments des zentralen, länglichen inneren Durchgangslochs (5) größer ist als der Innendurchmesser "c" des distalen Segments des zentralen, länglichen inneren Durchgangslochs (5) und das distale Segment und das proximale Segment des zentralen, länglichen inneren Durchgangslochs (5) in Fluidverbindung stehen;
- und wobei die Länge "h" des proximalen Endes des Verbindungsteils (3) mindestens 20 mm beträgt;
- wobei der Innendurchmesser "f" des proximalen Segments des inneren Durchgangslochs (5) des zylindrischen Verbindungsteils (3) 3,8 mm bis 5,2 mm beträgt, sich vom Innendurchmesser "c" des distalen Segments des zentralen, länglichen inneren Durchgangslochs (5) unterscheidet und ein Außendurchmesser "g" des zylindrischen Verbindungsteils (3) mindestens 7 mm beträgt;
- wobei sich das distale Segment mit dem Durchmesser "c" des zentralen, länglichen inneren Durchgangslochs (5) auf einer Seite der flexiblen Dichtungsmanschette (2) befindet und das proximale Segment mit dem Durchmesser "f" des zentralen, länglichen inneren Durchgangslochs (5) auf der anderen Seite der flexiblen Dichtungsmanschette (2) befindet;
- und wobei die Länge "a" des distalen Segments, das ein abgerundetes Spitzenteil (1) aufweist, von der Dichtungsmanschette (2) bis zum abgerundeten Spitzenteil (1), das in die äußere Harnröhrenöffnung einzuführen ist, 8 mm bis 12 mm beträgt; und
- der Außendurchmesser "b" des distalen Endes 6 mm (18 French) entsprechend einem durchschnittlichen Katheter beträgt;
- und wobei der Innendurchmesser "c" des distalen Segments des zentralen, länglichen inneren Durchgangslochs (5) 1,8 mm bis 3,5 mm beträgt, sich vom Innendurchmesser "f" des proximalen Segments des inneren Durchgangslochs des zylindrischen Verbindungsteils (3) unterscheidet;
- und wobei die flexible Dichtungsmanschette (2) des Adapters einen Durchmesser "d" von 12 mm bis 20 mm und eine Dicke "e" von 2 mm bis 5 mm aufweist.

2. Adapter nach Anspruch 1, **dadurch gekennzeichnet, dass** die Form des Endes des Spitzenteils (1), die konfiguriert ist, um in die äußere Harnröhrenöffnung eingeführt zu werden, zylindrisch oder konisch ist.

3. Adapter nach einem der Ansprüche 1 bis 2, **dadurch gekennzeichnet, dass** der Adapter ein Spritzenadapter ist und die Dosiervorrichtung eine Spritze ist.

4. Adapter nach einem der Ansprüche 1 bis 3, **dadurch gekennzeichnet, dass** das Verbindungsteil (4) der Dosiervorrichtung die Luer-Gleit-Spitze oder Luer-Verschluss-Spitze einer Spritze nach ISO 594 Standard ist.

5. Adapter nach einem der Ansprüche 1 bis 4, **dadurch gekennzeichnet, dass** das zylindrische Verbindungsteil (3) des Adapters (6) passgenau auf das Verbindungsteil (4) der Dosiervorrichtung passt, damit verbunden oder eingebaut ist oder Teil der Dosiervorrichtung ist.

6. Adapter nach einem der Ansprüche 1 bis 5, **dadurch gekennzeichnet, dass** das feste, pharmazeutisch anwendbare Material des Adapters ein für die Humantherapie anwendbares, flexibles Material ist und Kunststoff oder natürliches Material oder eine beliebige Legierung oder Mischung daraus ist, oder
duroplastischer, thermoplastischer oder flexibler Kunststoff oder duroplastisches Elastomer oder thermoplastisches Elastomer oder Latex oder auf Latex basierend oder synthetischer oder natürlicher Kautschuk oder auf Kautschuk basierend oder gummiähnliches Material oder auf gummiähnlichem Material basierendes Material oder eine beliebige Mischung daraus ist.

7. Adapter nach Anspruch 6 ist **dadurch gekennzeichnet, dass** der Kunststoff des pharmazeutisch anwendbaren, festen Materials des Adapters gefertigt ist aus: thermoplastischem Polyurethan, Polytetrafluorethylen (Teflon, PTFE), Polyethylen, Polypropylen, Polystyrol, Polyester, Polymilchsäure, Polycarbonat, Polyvinylchlorid, Polyethersulfon, Polyacrylat, Hydrogel (Acrylat), Polysulfon, Polyetheretherketon (PEEK), Poly-p-Xylylen (Parylen), Fluorpolymer oder Silikon oder einer beliebigen Mischung daraus.

8. Adapter nach einem der Ansprüche 6 bis 7, **dadurch gekennzeichnet, dass** das pharmazeutisch anwendbare, feste Material des Adapters ein für die Massenproduktion in Serie geeignetes Material ist, das für die Druckgusstechnologie oder den 3D-Druck geeignet ist.

9. Adapter nach einem der Ansprüche 1 bis 8 ist **dadurch gekennzeichnet, dass** an dem Ende des Spitzenteils (1), das konfiguriert ist, um in die äußere Harnröhre eingeführt zu werden, die Spitze des inneren Durchgangslochs (5) aufgeweicht sind, sodass keine scharfen Kanten verbleiben, die Schleimhautverletzungen verursachen, und das Abrunden und Aufweichen nach außen und innen in Richtung des inneren Durchgangslochs (5) ebenso erfolgen, sodass das abgerundete und aufgeweichte Spitzenteil (1) perfekt und im Allgemeinen an die äußere Harnröhrenöffnung sowohl männlicher als auch weiblicher Patienten passt.

10. Adapter nach einem der Ansprüche 1 bis 9 ist **dadurch gekennzeichnet, dass** die Länge "a" des Spitzenteils (1), das konfiguriert ist, um in die äußere Harnröhrenöffnung eingeführt zu werden, 8 mm oder 10 mm beträgt, und der Durchmesser "c" des zentralen, länglichen inneren Durchgangslochs (5) des distalen Segments, welches das Spitzenteil (1) aufweist, 2,5 mm oder 3,5 mm beträgt.

11. Adapter (6) nach einem der Ansprüche 1 bis 10, **dadurch gekennzeichnet, dass** der Innendurchmesser "f" des proximalen Segments des zentralen länglichen inneren Durchgangslochs (5) des zylindrischen Verbindungsteils (3) 4,2 mm oder 3,8 mm beträgt und der Außendurchmesser "g" des zylindrischen Verbindungsteils (3) 7 mm beträgt.

12. Adapter (6) nach einem der Ansprüche 1 bis 11, **dadurch gekennzeichnet, dass** die Länge "h" des proximalen Segments, welches das zylindrische Verbindungsteil (3) aufweist, 20 mm beträgt.

13. Adapter nach einem der Ansprüche 1 bis 12, **dadurch gekennzeichnet, dass** der Durchmesser "d" der flexiblen Dichtungsmanschette (2) 15 mm beträgt und die Dicke "e" 2 mm oder 3 mm beträgt.

14. Adapter nach einem der Ansprüche 1 bis 13, **dadurch gekennzeichnet, dass** die Kanten der Dichtungsmanschette (2) auf 45° abgeschrägte oder in einem Kreis mit 1 mm Radius abgerundet sind und die verbleibenden Kanten aufgeweicht sind.

15. Adapter nach einem der Ansprüche 1 bis 14, **dadurch gekennzeichnet, dass** der Außenumfang der Dichtungsmanschette (2) gerändelt (7) ist.

16. Adapter nach einem der Ansprüche 1 bis 15, **dadurch gekennzeichnet, dass** er zum einmaligen Gebrauch bestimmt ist.

17. Baugruppe, die eine Dosiervorrichtung und den Adapter (6) nach einem der vorstehenden Ansprüche umfasst, wobei die Dosiervorrichtung nach den Ansprüchen 1 und 5 passgenau auf den Adapter (6) passt, damit verbunden, verknüpft, integriert oder eingebaut ist.

## Revendications

1. Adaptateur (6) en matériau solide applicable pharmaceutiquement pour une instillation intravésicale, une injection de principe actif et/ou d'agent de contraste sous forme liquide, analogue à un fluide, gazeuse ou colloïdale dispersée dans la vessie par l'urètre, ***caractérisé en ce que***
- l'adaptateur (6) est destiné au remplacement d'un cathéter et convient à une injection indolore, non invasive et sans fuite dans l'urètre et la vessie par l'orifice urétral externe du patient, homme ou femme ;
- l'adaptateur (6) est pourvu d'un trou traversant intérieur longitudinal central (5), et
- comprenant une extrémité distale présentant une partie de pointe arrondie (1) configurée pour être insérée séparément et directement dans l'orifice urétral externe de patients masculins et féminins ;
- l'extrémité distale présentant un segment distal du trou traversant intérieur longitudinal central (5) avec un diamètre interne « c » constant et
- l'adaptateur (6) est réalisé en un matériau flexible et pourvu d'un collier d'étanchéité (2) avec un diamètre supérieur au diamètre de l'extrémité distale, dans lequel ledit collier d'étanchéité (2) s'adapte sur l'orifice urétral externe de forme grossièrement ronde ou ovale, et assure ainsi une étanchéité parfaite sans fuite et créant ainsi une surpression pour ouvrir le sphincter de la vessie chez les patients hommes et femmes et
- l'adaptateur (6) est pourvu d'une extrémité proximale présentant une partie de connexion cylindrique (3) pouvant être connectée à la partie de connexion (4) d'un dispositif de dosage, la partie de connexion cylindrique (3) présentant un segment proximal du trou traversant intérieur longitudinal central (5) avec un diamètre interne « f » et un diamètre externe « g » constants ;
- dans lequel le diamètre interne « f » du segment proximal du trou traversant intérieur longitudinal central (5) est plus grand que le diamètre interne « c » du segment distal du trou traversant intérieur longitudinal central (5), et le segment distal et le segment proximal du trou traversant intérieur longitudinal central (5) sont en communication fluidique ;
- et dans lequel la longueur « h » de l'extrémité proximale de la partie de connexion (3) est d'au moins 20 mm ;
- dans lequel le diamètre interne « f » du segment proximal du trou traversant intérieur (5) de la partie de connexion cylindrique (3) est de 3,8 mm à 5,2 mm différent du diamètre interne « c » du segment distal du trou traversant intérieur longitudinal central (5), et un diamètre extérieur « g » de la partie de connexion cylindrique (3) est au minimum de 7 mm ;
- avec le segment distal de diamètre « c » du trou traversant intérieur longitudinal central (5) se trouvant d'un certain côté du collier d'étanchéité flexible (2) et le segment proximal de diamètre « f » du trou traversant intérieur longitudinal central (5) se trouvant de l'autre côté du collier d'étanchéité flexible (2) ;
- et dans lequel la longueur « a » du segment distal présentant une partie de pointe arrondie (1) à partir du collier d'étanchéité (2) vers la partie de pointe arrondie (1) à insérer dans l'orifice urétral externe est de 8 mm à 12 mm ; et
- le diamètre extérieur « b » de l'extrémité distale est de 6 mm (18 French), correspondant à un cathéter moyen ;
- et dans lequel le diamètre interne « c » du segment distal du trou traversant intérieur longitudinal central (5) est de 1,8 mm à 3,5 mm différent du diamètre interne « f » du segment proximal du trou traversant intérieur de la partie de connexion cylindrique (3) ;
- et dans lequel le collier d'étanchéité flexible (2) de l'adaptateur présente un diamètre « d » de 12 mm à 20 mm, et une épaisseur « e » de 2 mm à 5 mm.

2. Adaptateur selon la revendication 1, ***caractérisé en ce que*** la forme de l'extrémité de la partie de pointe (1) configurée pour être insérée dans l'orifice urétral externe est cylindrique ou conique.

3. Adaptateur selon l'une quelconque des revendications 1 à 2, ***caractérisé en ce que*** l'adaptateur est un adaptateur de seringue et le dispositif de dosage est une seringue.

4. Adaptateur selon l'une quelconque des revendications 1 à 3, ***caractérisé en ce que*** la partie de connexion (4) du dispositif de dosage est l'embout Luer Slip ou l'embout Luer Lock standard ISO 594 d'une seringue.

5. Adaptateur selon l'une quelconque des revendications 1 à 4, ***caractérisé en ce que*** la partie de connexion cylindrique (3) de l'adaptateur (6) s'adapte précisément, est connectée à ou intégrée à la partie de connexion (4) du dispositif de dosage ou fait partie du dispositif de dosage.

6. Adaptateur selon l'une quelconque des revendications 1 à 5, ***caractérisé en ce que*** le matériau applicable pharmaceutiquement solide de l'adaptateur est un matériau flexible applicable à la thérapie humaine et est un matériau à base de plastique ou de matière naturelle ou de tout alliage ou mélange de ceux-ci, ou
est un matériau thermodurcissable, thermoplastique ou plastique flexible ou un élastomère thermodurci ou un élastomère thermoplastique ou un latex ou à base de latex, ou du caoutchouc ou un matériau à base de caoutchouc ou un matériau analogue à du caoutchouc naturel ou synthétique, ou un matériau à base de matériaux analogue à du caoutchouc, ou tout mélange de ceux-ci.

7. Adaptateur selon la revendication 6, ***caractérisé en ce que*** le plastique dont est fait le matériau applicable pharmaceutiquement solide de l'adaptateur est : polyuréthane thermoplastique, polytétrafluoroéthylène (Téflon, PTFE), polyéthylène, polypropylène, polystyrène, polyester, acide polylactique, polycarbonate, chlorure de polyvinyle, polyéthersulfone, polyacrylate, hydrogel (acrylate), polysulfone, polyétheréthercétone (PEEK), poly-p-xylylène (parylène), fluoropolymère, ou silicone ou tout mélange de ceux-ci.

8. Adaptateur selon l'une quelconque des revendications 6 à 7, ***caractérisé en ce que*** le matériau applicable pharmaceutiquement solide de l'adaptateur est un matériau destiné à une production de masse en série, adapté à la technologie de moulage sous pression ou à l'impression 3D.

9. Adaptateur selon l'une quelconque des revendications 1 à 8, ***caractérisé en ce qu'**à* l'extrémité de la partie de pointe (1) configurée pour être insérée dans l'urètre externe, la pointe du trou traversant intérieur (5) est adoucie de sorte qu'aucun bord tranchant provoquant une lésion de la muqueuse ne reste et l'arrondi et l'adoucissement sont également réalisés vers l'extérieur et vers l'intérieur en direction du trou traversant intérieur (5) et ainsi la partie de pointe (1) arrondie et adoucie s'adapte parfaitement et de manière générale à l'orifice urétral externe des patients hommes et femmes.

10. Adaptateur selon l'une quelconque des revendications 1 à 9, ***caractérisé en ce que*** la longueur « a » de la partie de pointe (1) configurée pour être insérée dans l'orifice urétral externe est de 8 mm ou 10 mm, le diamètre « c » du trou traversant intérieur longitudinal central (5) du segment distal présentant la partie de pointe (1) est de 2,5 mm ou 3,5 mm.

11. Adaptateur (6) selon l'une quelconque des revendications 1 à 10, ***caractérisé en ce que*** le diamètre interne « f » du segment proximal du trou traversant intérieur longitudinal central (5) de la partie de connexion cylindrique (3) est de 4,2 mm ou de 3,8 mm et le diamètre extérieur « g » de la partie de connexion cylindrique (3) est de 7 mm.

12. Adaptateur (6) selon l'une quelconque des revendications 1 à 11, ***caractérisé en ce que*** la longueur « h » du segment proximal présentant la partie de connexion cylindrique (3) est de 20 mm.

13. Adaptateur selon l'une quelconque des revendications 1 à 12, ***caractérisé en ce que*** le diamètre « d » du collier d'étanchéité flexible (2) est de 15 mm, et l'épaisseur « e » est de 2 mm ou 3 mm.

14. Adaptateur selon l'une quelconque des revendications 1 à 13, ***caractérisé en ce que*** les bords du collier d'étanchéité (2) sont chanfreinés à 45° ou arrondis dans un cercle avec un rayon de 1 mm, et les bords restants sont adoucis.

15. Adaptateur selon l'une quelconque des revendications 1 à 14, ***caractérisé en ce que*** la circonférence extérieure du collier d'étanchéité (2) est moletée (7).

16. Adaptateur selon l'une quelconque des revendications 1 à 15, ***caractérisé en ce* qu'**il est à usage unique.

17. Ensemble comprenant un dispositif de dosage et l'adaptateur (6) selon l'une quelconque des revendications précédentes, dans lequel le dispositif de dosage s'adapte précisément, est connecté, lié, intégré ou incorporé à l'adaptateur (6) selon les revendications 1 et 5.
